# FASCICULE DE BREVET EUROPEEN

(11) **EP 0 720 493 B1**
(45) Date de publication et mention de la délivrance du brevet: **02.06.1999**
(21) Numéro de dépôt: 94922286.3
(22) Date de dépôt: 21.07.1994
(51) Int. Cl.: A61M 16/00, A61M 16/04, A62B 9/06, B63C 11/18

(54) **EMBOUT RESPIRATOIRE INTRA-BUCCAL ANATOMIQUE**
ANATOMISCHES, INTRABUCCALES ATMUNGSANSCHLUSSSTÜCK
ANATOMICAL INTRA-BUCCAL BREATHING MOUTHPIECE

(43) Date de publication de la demande: 10.07.1996
(73) Titulaire: David, Michel, 69004 Lyon (FR)
(72) Inventeur: David, Michel, 69004 Lyon (FR)
(74) Mandataire: Vuillermoz, Bruno
(86) Numéro de dépôt international: FR9400912
(87) Numéro de publication internationale: WO9603173

(56) Documents cités:
- GB-A- 699 950
- US-A- 3 107 667
- US-A- 4 136 689
- US-A- 4 466 434
- US-A- 4 495 945
- US-A- 5 031 611
- FERNER H. & STAUBESAND J. 'Sobotta Atlas d'Anatomie Humaine' 1985 , URBAN & SCHWARZENBERG , DE, MüNCHEN voir figure 302

## Description

### Domaine technique

L'invention concerne un embout respiratoire intra-buccal anatomique ; elle se rapporte plus particulièrement à un embout respiratoire anatomique à usage médical, par exemple pour des malades "frappés d'insuffisance respiratoire", c'est-à-dire qui ne peuvent plus respirer par eux-mêmes, en continu ou temporairement, quelle qu'en soit la cause, tels que des patients atteints de myopathie, de tuberculose ou également pour les traitements par aérosols médicamenteux ou non, de malades contagieux (VIH positif, etc ...). Elle concerne également un embout respiratoire pour la plongée subaquatique.

Dans la suite de la description, l'invention sera plus particulièrement décrite dans son application médicale.

### Etat de la technique

Pour aider les insuffisants respiratoires à respirer, le plus généralement, on leur insère dans la bouche un dispositif tronconique aplati présentant d'un côté un premier embout circulaire extra-buccal destiné à être raccordé à la source d'air renforcé en oxygène, et un second embout intra-buccal dont l'orifice de sortie est aplati et comporte un léger rebord pour être retenu par les dents du patient. Bien que très largement utilisé, ce dispositif présente de nombreux inconvénients, à savoir notamment :
- un manque de stabilité en bouche,
- un manque d'étanchéité,
- des rejets intempestifs par le patient, notamment la nuit ou lors d'insufflations d'air,
   inconvénients qui nécessitent une surveillance quasi-permanente de ces patients.

Pour pallier ces inconvénients, on aurait pu penser à équiper l'extrémité intra-buccale de ce dispositif d'une orthèse appropriée. Malheureusement la chose n'est pas envisageable, car le moulage des maxillaires respectivement supérieur et inférieur et des mandibules, ainsi que l'enregistrement de l'occlusion, ne sont malheureusement pas possibles compte tenu de l'état gravissime de ces patients.

De la sorte, malgré les inconvénients rappelés ci-dessus, la technique du dispositif aplati continue à être largement utilisée.

Dans les cas extrêmes, si cette technique n'est pas supportée, ou est rejetée, il faut procéder soit à des intubations nasales, soit à une trachéotomie.

Dans le document GB-A-699 950, on a décrit un embout respiratoire pour des patients destinés à être soumis à des électro-chocs, comprenant une partie moulée recevant les deux mâchoires et destinée à les maintenir séparées. Ce dispositif destiné à un usage spécifique (électrochocs) présente un tuyau rigide qui pénètre dans la bouche et couvre une grande partie de la langue, ce qui occasionne des gênes considérables. De plus, il oblige à l'avancée mandibulaire et n'autorise donc pas un port prolongé confortable.

### Description de l'invention

L'invention pallie ces inconvénients. Elle vise un dispositif respiratoire intrabuccal, notamment pour les patients nécessitant une assistance respiratoire, qui soit facile à fabriquer, économique, ne présente pas les inconvénients énumérés ci-dessus, et puisse être très facilement mis en place, sans avoir à prendre préalablement des empreintes ou analogues, et ce quel que soit l'état ou la conformation du patient.

Cet embout respiratoire intra-buccal anatomique qui comprend deux parties, à savoir :
- une première partie extra orale présentant un premier embout extra-buccal destiné à être raccordé à la source de gaz, et un second embout intra-buccal dont l'orifice de sortie est aplati :
- une seconde partie formant un élément souple, associé à l'embout intra-buccal comprenant :
   . des parties vestibulaires verticales épousant la forme anatomique des vestibules, respectivement supérieure et inférieure ;
   . des rebords linguaux verticaux, respectivement supérieur et inférieur ;
   . un tampon vestibulo-lingual inter-occlusal plan reliant les parties vestibulaires et les rebords, disposé horizontalement à la jonction entre les parties supérieure et inférieure, l'épaisseur dudit tampon plan étant légèrement supérieure à celle de l'intervalle inter-occlusal des mâchoires en position de repos ;
   se **caractérise** :
- en ce que l'orifice de sortie aplati intra-buccal, débouche dans l'épaisseur du tampon vestibulo-lingual ;
- et en ce que la partie vestibulaire supérieure disposée dans le sens antéropostérieur, est située en avant de la partie vestibulaire inférieure.

Dans la description suivante l'élément souple est designé comme une orthèse.

En d'autres termes, l'invention consiste dans un embout respiratoire du type en question, d'une part à ménager une orthèse souple avec vestibules et rebords linguaux verticaux reliés par un tampon plan horizontal, et dont l'implantation des vestibules prend en compte la position mandibulaire moyenne par rapport au maxillaire de manière à éviter le proglissement mandibulaire et ainsi améliorer le confort, et d'autre part, à prévoir que l'orifice de sortie aplati intra-buccal débouche dans le tampon plan, de manière à ne pas causer de gène au niveau de la langue.

De la sorte, on réalise un dispositif standard susceptible de tenir compte de la morphologie propre de chaque patient, et qui peut donc être utilisé immédiatement et avec efficacité sur n'importe quel type de patient nécessitant une assistance respiratoire, quelle qu'en soit la cause.

Avantageusement, en pratique :
- l'orifice de sortie aplati intra-buccal débouche en partie avant ou dans les parties latérales voire postérieures du tampon vestibulo-lingual ;
- la distance entre les deux parties vestibulaires supérieure et inférieure est de l'ordre de deux à quatre millimètres, avantageusement voisine de trois millimètres ;
- l'épaisseur des parties vestibulaires verticales décroit depuis la base de raccordement au tampon plan jusqu'à leur extrémité supérieure ; dans une forme de réalisation pratique, l'épaisseur des vestibules verticaux est d'environ deux millimètres à la base et d'un millimètre à l'extrémité libre ;
- le contour libre de la partie vestibulaire supérieure épouse le contour des brides et des freins de la mâchoire supérieure ; de la sorte, il est facile avec une paire de ciseaux, de modifier ce contour dans le cas de vestibules peu profonds ou de freins hypertrophiques ;
- l'épaisseur du tampon varie de trois à quatre millimètres, et la largeur du plan de la portion vestibulo-linguale est comprise selon le modèle entre dix et quinze millimètres, de manière à être légèrement supérieure à l'épaisseur des dents ; de la sorte, on peut aisément réaliser plusieurs modèles, l'un dans lequel le tampon a une largeur d'environ dix millimètres, un autre d'une largeur moyenne de douze millimètres et un autre d'une largeur supérieure de quinze millimètres ;
- les rebords linguaux ont une épaisseur légèrement dégressive à partir de la portion de jonction, par exemple de 2 à 1,5 mm ;
- les parties vestibulaires et les rebords s'arrêtent sensiblement au niveau des secondes prémolaires ;
- la première partie extra-orale forme un angle de 15 à 30°, de préférence au voisinage de 20° par rapport au plan d'occlusion ; cette inclinaison présente l'avantage d'éviter que le tuyau de raccordement à la source de gaz, d'air par exemple, à l'embout extra-buccal pointe verticalement vers le haut lorsque le patient est allongé ; ainsi, cette tuyauterie est moins vulnérable ;
- l'orthèse est réalisée en un matériau thermoplastique biocompatible souple thermoformable, notamment à la température du corps humain au niveau de la bouche, de manière à ce que cette orthèse intra-buccale s'auto-adapte rapidement et progressivement au volume buccal réel du patient, voire rapidement par immersion dans de l'eau chaude (45°C-50°C); on utilise par exemple du chlorure de polyvinyle (PVC) fortement plastifié, mais non chargé, pour obtenir une grande souplesse ou tout autre matériau présentant les mêmes caractéristiques physiques.

La manière dont l'invention peut être réalisée et les avantages qui en découlent, ressortiront mieux de l'exemple de réalisation qui suit à l'appui des figures annexées.

### Brève description des dessins

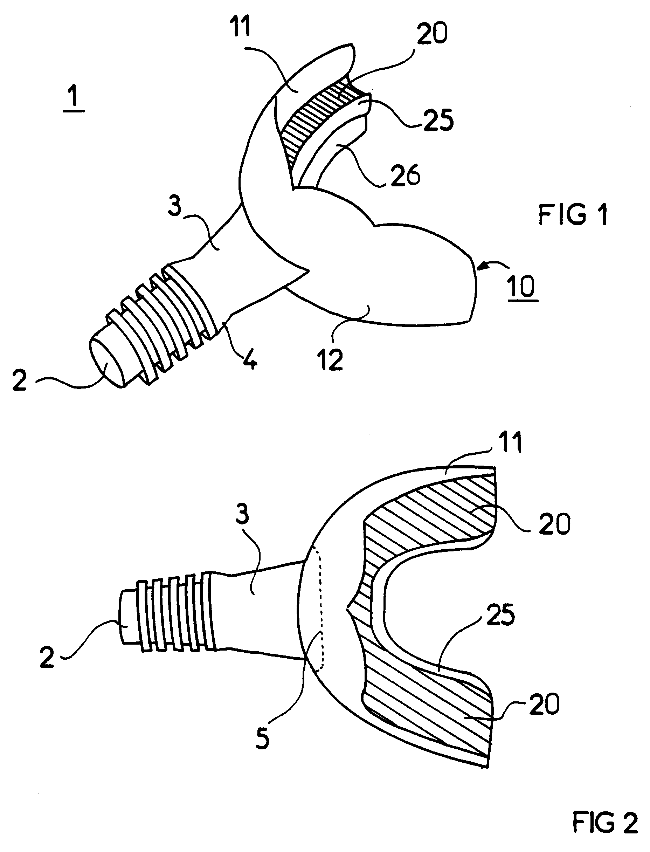

La figure 1 est une représentation en perspective sommaire vue trois quarts avant d'un embout respiratoire conforme à l'invention.
La figure 2 est une vue de dessus de cet embout monté en section longitudinale horizontale à la figure 3.
La figure 4 est une vue de détail du raccordement de la première partie extra orale sur la seconde partie.
La figure 5 est une représentation en perspective sommaire troisquart arrière d'une autre forme d'exécution de l'invention, montrée en vue arrière à la figure 6.

### Manière de réaliser l'invention

En se référant aux figures, l'embout respiratoire intra-buccal anatomique conforme à l'invention, comprend essentiellement deux parties solidarisées entre elles, par exemple venues de moulage ou thermosoudées, à savoir respectivement une première partie désignée par la référence générale (1) et une seconde partie par la référence générale (10). Il va de soi que ces deux parties principales peuvent être elles-même constituées de plusieurs parties secondaires.

La première partie (1) extra-orale, connue par ailleurs qui, correspondant à l'état antérieur de la technique, comprend un premier embout cylindrique (2) extra-buccal, destiné à être raccordé à la source de gaz, par exemple d'air enrichi en oxygène, puis un corps (3) en forme de tronc de pyramide aplati raccordé à l'embout (2) par une portion tronconique (4). Ce corps (3) se termine par un second embout intra -buccal (5) dont l'orifice de sortie (6) est aplati, par exemple présente une section rectangulaire de vingt millimètres sur deux millimètres.

La première partie de l'embout (1) est associée à une seconde partie (10) formant une orthèse souple, par exemple en PVC fortement plastifiée. Cette seconde partie (10) comprend tout d'abord deux vestibules verticaux, respectivement supérieure (11) et inférieure (12), dont la forme épouse la forme anatomique des vestibules.

Dans une forme de réalisation pratique, la base (13,14) de raccordement de ces vestibules (11,12) à l'orifice de sortie intra-buccal (5,6), a une épaisseur voisine de deux millimètres, alors que l'extrémité libre (15,16) a une épaisseur voisine de un millimètre.

La seconde partie (10) comporte également, disposé horizontalement à la jonction des parties supérieure (11) et inférieure (12), un tampon vestibulo-lingual plan désigné par la référence (20) en forme de trottoir horizontal lisse voire strié inter-occlusal reliant les vestibules (11,12) aux deux rebords linguaux (25,26) essentiellement verticaux, respectivement supérieur (25) et inférieur (26).

Selon une autre caractéristique importante de l'invention, l'épaisseur du tampon (20) plan horizontal est légèrement supérieure à l'intervalle inter-occlusal des mâchoires en position de repos, ce qui autorise un bon maintien en bouche sans effort et sans contracture musculaire, les dents reposant sur le tampon (20).

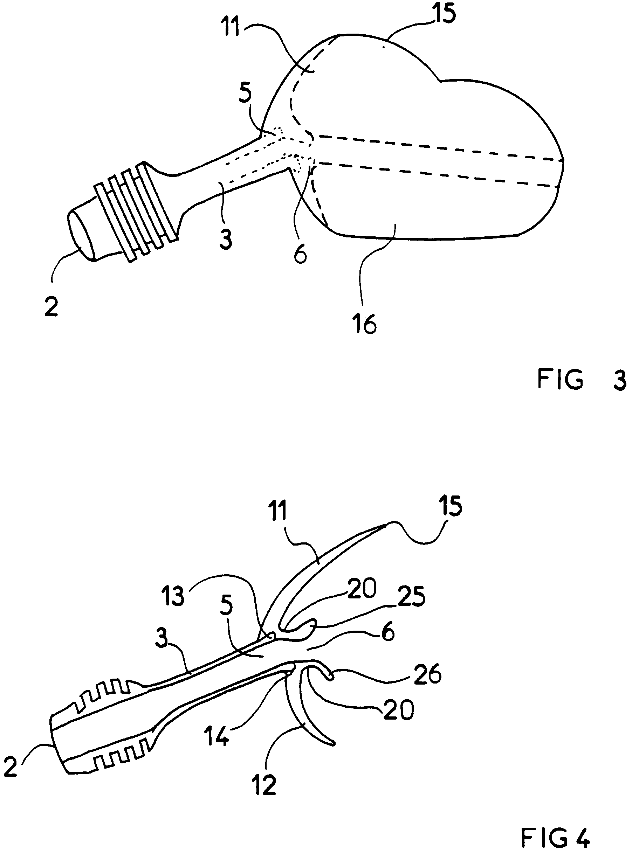

De même, selon une caractéristique importante de l'invention, l'orifice de sortie aplati (6) intra-buccal (5), débouche dans l'épaisseur du tampon (20) vestibulo-lingual (voir figure 4).

Dans une variante, l'embout de l'invention peut être également utilisé dans les prognathismes importants par retournement et découpes appropriées

Il peut être également utilisé dans une situation d'urgence, pour provoquer l'avancée de la mandibule de manière à dégager les voies aériennes.

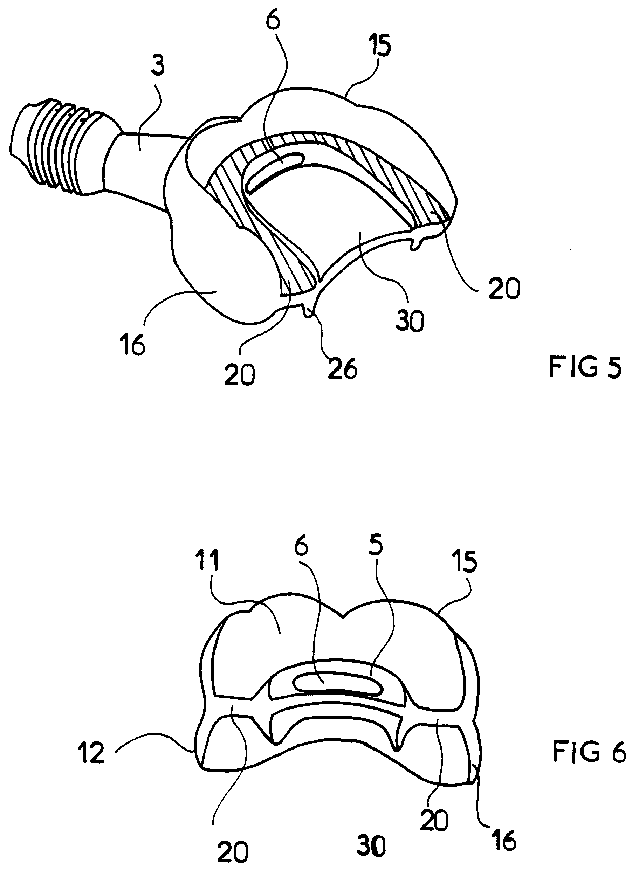

Dans une autre variante, montrée à la figure 5, les deux parties du tampon intraocclusal (20) sont reliés entre eux par une languette horizontale légèrement bombée (30), destiné à immobiliser la langue pour éviter le risque d'obturer l'orifice bucco-pharingé.

Le dispositif selon l'invention présente de nombreux avantages par rapport à ceux commercialisés à ce jour et rappelés dans le préambule. On peut citer :
- la possibilité de réaliser grâce à un dispositif standard un nombre restreint de modèles, par exemple petits, moyens et grands voire néonatals, sans avoir à prendre des empreintes, dont on sait qu'elles sont impossibles à saisir sur ces patients ;
- la possibilité de disposer des dispositifs immédiatement utilisables, notamment en cas de crise des patients ;
- l'auto-adaptabilité au volume buccal du patient ;
- une bonne stabilité en bouche, évitant des pertes accidentelles notamment pendant le sommeil ou pendant un coma ;
- une excellente étanchéité ;
- l'absence de rejet intempestif ;
- le fait que le patient n'a pas d'effort à fournir pour maintenir le dispositif en bouche ;
- la facilité avec laquelle on peut modifier le contour pour mieux se conformer à la morphologie du patient.

De la sorte, ce dispositif peut être utilisé avec succès pour toutes les insuffisances respiratoires médicales de toutes natures, notamment lorsque l'on recherche un dispositif à usage unique.

Il peut être également utilisé chez des patients édentés totalement ou partiellement.

Ce dispositif peut être également utilisé avec succès pour les embouts de plongées subaquatique, car il évite le proglissement mandibulaire, générateur à la longue, de douleurs de l'articulation temporo-mandibulaire. Dans cette application, la partie extra-orale (1) est raccordable directement au détendeur ou au tube de respiration de surface, dénommé communément "tuba".

## Revendications

1. Embout respiratoire intra-buccal anatomique en deux parties, à savoir :
- une première partie (1) extra orale présentant un premier embout (2) extra-buccal destiné à être raccordé à la source de gaz, et un second embout (5) intra-buccal dont l'orifice de sortie est aplati (6) :
- une seconde partie (10) formant un élément souple, associé à l'embout intra-buccal (5) comprenant :
. des parties vestibulaires verticales (11,12) épousant la forme anatomique des vestibules, respectivement supérieure (11) et inférieure (12) ;
. des rebords linguaux, verticaux, respectivement supérieur (25) et inférieur (26) ;
. un tampon vestibulo-lingual (20) inter-occlusal plan reliant les parties vestibulaires (11,12) et les rebords (25,26), disposé horizontalement à la jonction entre les parties supérieure et inférieure, l'épaisseur dudit tampon (20) plan étant légèrement supérieure à celle de l'intervalle inter-occlusal des mâchoires en position de repos ;
**caractérisé** :
- en ce que l'orifice de sortie (6) aplati intra-buccal (5), débouche dans l'épaisseur du tampon (20) vestibulo-lingual ;
- et en ce que la partie vestibulaire supérieure (11) disposée dans le sens antéropostérieur, est située en avant de la partie vestibulaire inférieure (12).

2. Embout respiratoire selon la revendication 1, caractérisé en ce que l'orifice de sortie (6) aplati intra-buccal (5), débouche à l'avant ou dans les parties latérales ou postérieures du tampon (20) vestibulo-lingual.

3. Embout respiratoire selon l'une des revendications 1 à 2, caractérisé en ce que la distance entre les deux parties vestibulaires supérieure (11) et inférieure (12), est comprise entre deux et quatre millimètres.

4. Embout respiratoire selon l'une des revendications 1 à 3, caractérisé en ce que l'épaisseur des parties vestibulaires (11,12) décroît depuis la base (13,14) de raccordement au tampon (20) jusqu'à leur extrémité (15,16).

5. Embout respiratoire selon l'une des revendications 1 à 4, caractérisé en ce que le contour libre de la partie vestibulaire supérieure (11) épouse le contour des brides et des freins de la mâchoire supérieure (6).

6. Embout respiratoire selon l'une des revendications 1 à 5, caractérisé en ce que l'épaisseur du tampon vestibulo-lingual (20) horizontal est comprise entre trois et quatre millimètres, et en ce que la largeur de ce tampon (20) plan est comprise entre dix et quinze millimètres.

7. Embout respiratoire selon l'une des revendications 1 à 6, caractérisé en ce que la première partie (1) forme un angle de 15 à 30° par rapport au plan de d'occlusion.

8. Embout respiratoire selon l'une des revendications 1 à 7, caractérisé en ce que la seconde partie (10) est réalisée en matière thermoplastique biocompatible souple thermoformable à la température du corps humain au niveau de la bouche.

9. Embout respiratoire selon l'une des revendications 1 à 8, caractérisé en ce qu'il est destiné à un usage médical.

10. Embout respiratoire selon l'une des revendications 1 à 8, caractérisé en ce qu'il est destiné à la plongée subaquatique.

## Claims

1. Anatomical intrabuccal respiratory mouthpiece in two parts, namely :
- a first extraoral part (1) having a first extrabuccal connection piece (2) intended to be joined to the gas source, and a second intrabuccal connection piece (5) whose outlet orifice is oblate (6) ;
- a second part (10) forming a flexible element, associated with the intrabuccal connection piece (5), including :
vertical vestibular parts (11, 12) matching the anatomical shape of the vestibules, being the upper (11) and lower (12) vestibules, respectively ;
upper (25) and lower (26) vertical lingual rims, respectively ;
a plane interocclusal vestibulo-lingual pad (20) which joins the vestibular parts (11, 12) and the rims (25, 26) and is arranged horizontally at the junction between the upper and lower parts, the thickness of said plane pad (20) being slightly greater than that of the interocclusal gap of the jaws in the rest position ;
characterized :
- in that the oblate intrabuccal (5) outlet orifice (6) opens out within the thickness of the vestibulo-lingual pad (20) ;
- and in that the upper vestibular part (11) arranged in the anteroposterior direction is situated in front of the lower vestibular part (12).

2. Respiratory mouthpiece according to Claim 1, characterized in that the oblate intrabuccal (5) outlet orifice (6) opens out at the front, or in the lateral or posterior parts, of the vestibulo-lingual pad (20).

3. Respiratory mouthpiece according to one of Claims 1 and 2, characterized in that the distance between the two upper (11) and lower (12) vestibular parts is between two and four millimeters.

4. Respiratory mouthpiece according to one of Claims 1 to 3, characterized in that the thickness of the vestibular parts (11, 12) decreases from the base (13, 14), connecting with the pad (20), to their end (15, 16).

5. Respiratory mouthpiece according to one of Claims 1 to 4, characterized in that the free contour of the upper vestibular part (11) matches the contour of the bridles and frenula of the upper jaw (6).

6. Respiratory mouthpiece according to one of Claims 1 to 5, characterized in that the thickness of the horizontal vestibulo-lingual pad (20) is between three and four millimeters, and in that the width of this plane pad (20) is between ten and fifteen millimeters.

7. Respiratory mouthpiece according to one of Claims 1 to 6, characterized in that the first part (1) forms an angle of 15 to 30° in relation to the occlusion plane.

8. Respiratory mouthpiece according to one of Claims 1 to 7, characterized in that the second part (10) is made of biocompatible and flexible thermoplastic material which is thermoformable at the body temperature in the mouth area.

9. Respiratory mouthpiece according to one of Claims 1 to 8, characterized in that it is intended for a medical use.

10. Respiratory mouthpiece according to one of Claims 1 to 8, characterized in that it is intended for skin diving.

## Patentansprüche

1. Anatomisches intrabuccales Atmungsanschlußstück aus zwei Teilen, nämlich:
- einem ersten Teil (1) außerhalb des Mundes mit einem ersten, extrabuccalen Anschlußstück (2), welches zum Anschluß an die Gasquelle bestimmt ist, und mit einem zweiten, intrabuccalen Anschlußstück (5), dessen Austrittsöffnung (6) abgeflacht ist;
- einem zweiten Teil (10), welcher ein biegsames, mit dem intrabuccalen Anschlußstück (5) verbundenes Element bildet und folgendes umfaßt:
· vertikale vestibulare Teile (11,12), welche die anatomische Form des oberen (11) bzw. unteren (12) Vestibulums annehmen;
· eine obere (25) und eine untere (26) vertikale, linguale Randleiste;
· ein flaches inter-okklusales, vestibulo-linguales Kissen (20), das die vestibularen Teile (11,12) und die Randleisten (25,26) miteinander verbindet und horizontal an der Verbindungsstelle zwischen den oberen und unteren Teilen angeordnet ist, wobei die Dicke des genannten flachen Kissens (20) etwas größer ist als die des inter-okklusalen Zwischenraums der Kiefer in Ruhestellung;
**dadurch gekennzeichnet:**
- daß die abgeflachte intrabuccale (5) Austrittsöffnung (6) in die Materialstärke des vestibulo-lingualen Kissens (20) mündet;
- und daß der obere, in anteroposteriorer Richtung angeordnete Teil (11) sich vor dem unteren vestibularen Teil (12) befindet.

2. Atmungsanschlußstück nach Anspruch 1, **dadurch gekennzeichnet**, daß die abgeflachte intrabuccale (5) Austrittsöffnung (6) in das Vorderteil oder in die lateralen oder posterioren Abschnitte des vestibulo-lingualen Kissens (20) mündet.

3. Atmungsanschlußstück nach einem der Ansprüche 1 bis 2, **dadurch gekennzeichnet**, daß der Abstand zwischen dem oberen (11) und dem unteren (12) vestibularen Teil zwischen zwei und vier Millimeter beträgt.

4. Atmungsanschlußstück nach einem der Ansprüche 1 bis 3, **dadurch gekennzeichnet**, daß die Dicke der vestibularen Teile (11,12) von der mit dem Kissen (20) verbundenen Basis (13,14) zu ihrem Ende (15,16) hin abnimmt.

5. Atmungsanschlußstück nach einem der Ansprüche 1 bis 4, **dadurch gekennzeichnet**, daß die freie Kontur des oberen vestibularen Teils (11) sich an die Kontur der medialen und lateralen Lippenbändchen des Oberkiefers (6) anpaßt.

6. Atmungsanschlußstück nach einem der Ansprüche 1 bis 5, **dadurch gekennzeichnet**, daß die Dicke des horizontalen vestibulo-lingualen Kissens (20) zwischen drei und vier Millimeter beträgt und daß die Breite dieses flachen Kissens (20) zwischen zehn und fünfzehn Millimeter beträgt.

7. Atmungsanschlußstück nach einem der Ansprüche 1 bis 6, **dadurch gekennzeichnet**, daß der erste Teil (1) einen Winkel von 15 bis 30° in bezug auf die Okklusionsebene bildet.

8. Atmungsanschlußstück nach einem der Ansprüche 1 bis 7, **dadurch gekennzeichnet**, daß der zweite Teil (10) aus einem biologisch verträglichen, biegsamen und bei der im Bereich des Mundes herrschenden menschlichen Körpertemperatur wärmeformbaren thermoplastischen Material hergestellt ist.

9. Atmungsanschlußstück nach einem der Ansprüche 1 bis 8, **dadurch gekennzeichnet**, daß es für eine medizinische Verwendung bestimmt ist.

10. Atmungsanschlußstück nach einem der Ansprüche 1 bis 8, **dadurch gekennzeichnet**, daß es zur Verwendung beim Unterwassertauchen bestimmt ist.
